(19) Europäisches Patentamt
European Patent Office
Office européen des brevets

(11) **EP 3 928 733 B1**

(12) **FASCICULE DE BREVET EUROPEEN**

(45) Date de publication et mention
de la délivrance du brevet:
**06.03.2024 Bulletin 2024/10**

(21) Numéro de dépôt: **21180679.9**

(22) Date de dépôt: **21.06.2021**

(51) Classification Internationale des Brevets (IPC):
**A61C 5/44** *(2017.01)* **A61K 6/52** *(2020.01)*
**A61K 6/56** *(2020.01)*

(52) Classification Coopérative des Brevets (CPC):
**A61C 5/44; A61K 6/52; A61K 6/56**

(54) **DISPOSITIF DE LOCALISATION D'APEX POUR MESURER LA CONCENTRATION D'UNE SOLUTION D'IRRIGATION DANS LA ZONE APICALE D'UN CANAL DE RACINE DENTAIRE**

APEX-LOKALISIERUNGSVORRICHTUNG ZUR MESSUNG DER KONZENTRATION EINER SPÜLLÖSUNG IN DER APIKALEN ZONE EINES ZAHNWURZELKANALS

APEX LOCATING DEVICE FOR MEASURING THE CONCENTRATION OF AN IRRIGATION SOLUTION IN THE APICAL AREA OF A DENTAL ROOT CANAL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **22.06.2020 FR 2006486**

(43) Date de publication de la demande:
**29.12.2021 Bulletin 2021/52**

(73) Titulaire: **Maillard, Thierry**
**33185 Le Haillan (FR)**

(72) Inventeur: **Maillard, Thierry**
**33185 Le Haillan (FR)**

(74) Mandataire: **Ipside**
**6, Impasse Michel Labrousse**
**31100 Toulouse (FR)**

(56) Documents cités:
**WO-A1-2008/114244 JP-A- 2007 229 110**
**JP-A- 2019 025 058**

## Description

### Domaine technique

La présente invention appartient au domaine général de l'endodontie, notamment des dispositifs de localisation d'apex, communément appelés localisateurs (électroniques) d'apex, et concerne plus particulièrement un localisateur d'apex permettant de mesurer la concentration de la solution d'irrigation dans le canal radiculaire avant la jonction cémento-dentinaire.

Un tel localisateur d'apex est par exemple utilisé lors de l'étape de préparation canalaire du traitement endodontique.

### Etat de l'art

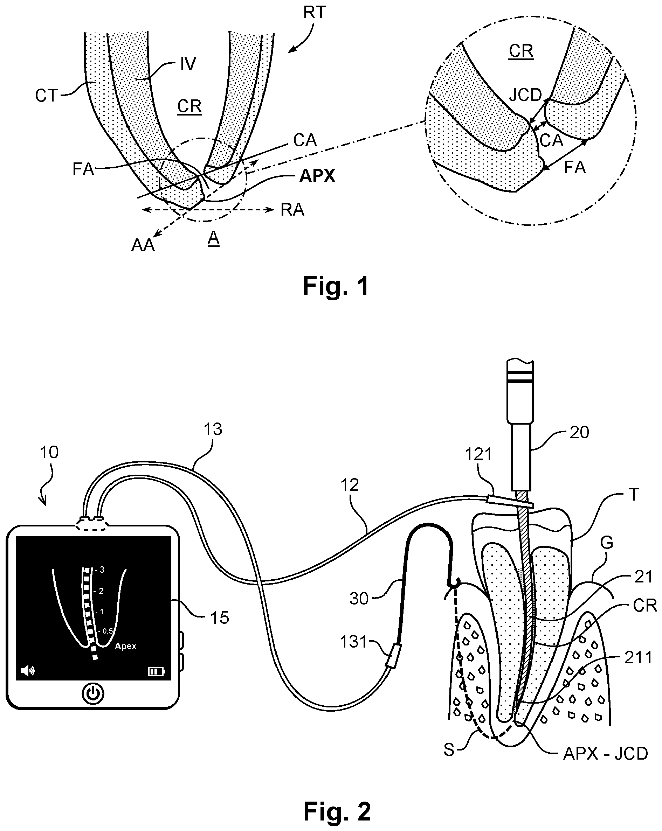

Il convient au préalable de rappeler brièvement l'anatomie d'une racine dentaire au niveau de la zone apicale. Le système canalaire et plus particulièrement la structure apicale peuvent par exemple être décrits selon le concept apical de *Kuttler* qui en donne un modèle assez représentatif schématisé en figure 1.

La figure 1 représente, en coupe longitudinale partielle, une racine dentaire RT traversée en son centre d'un canal radiculaire CR qui se termine par un orifice FA, dit foramen apical, à l'extrémité de ladite racine pour le passage des vaisseaux sanguins et des faisceaux nerveux. Le foramen apical FA présente généralement une forme de goulot d'étranglement due à un rétrécissement puis un élargissement du canal radiculaire CR qui définissent une zone de diamètre minimum dite constriction apicale CA. La constriction apicale CA constitue le passage le plus étroit du paquet vasculonerveux irriguant la pulpe. Globalement, le canal radiculaire CR présente une forme convergente puis divergente entre la partie coronaire et le péri-apex, qui s'apparente à deux cônes opposés par leurs sommets au niveau de la constriction apicale CA. Plus précisément, le canal radiculaire CR définit un cône dentinaire et un cône cémentaire plus court, l'interface entre les deux est appelée jonction cémento-dentinaire JCD. Comme représenté sur le détail A de la figure 1, la constriction apical CA et la jonction cémento-dentinaire JCD ne sont pas nécessairement confondues, mais il arrive qu'elles le soient. La littérature spécialisée montre que la CA coïncide régulièrement avec la JCD ou se trouve à proximité de celle-ci.

Lors d'un traitement endodontique, le praticien (chirurgien-dentiste) cherche à éliminer tous les matériaux, débris et fluides organiques qui emplissent le canal radiculaire jusqu'au fond, c'est-à-dire jusqu'à au foramen apical, pour éviter une réapparition d'un abcès dentaire dans ledit canal. Toutefois, son objectif est surtout, autant que possible, de ne pas dépasser le terminus apical APX, ou apex, d'une part, pour ne pas occasionner de douleur au patient, et d'autre part, pour ne pas creuser une cavité sous la racine, au-delà de l'apex, ce qui pourrait donner lieu à l'apparition d'un abcès.

Il est donc primordial pour le praticien de localiser très précisément le foramen apical et l'apex, notamment lors des opérations de nettoyage et de mise en forme du canal radiculaire (préparation canalaire), pour éviter de franchir le foramen apical. Cela revient à déterminer « la longueur de travail » qui désigne la longueur exacte entre un repère coronaire et une limite apicale fixée et choisie à l'avance, classiquement située à la constriction apicale. Cette longueur de travail peut être déterminée à l'aide d'une radiographie (qui donne l'apex radiographique RA moins précis) et/ou d'un localisateur électronique d'apex (qui donne l'apex anatomique AA plus précis).

La localisation d'apex permet également de bien conduire l'irrigation, qui est une étape clé de la préparation canalaire.

L'objectif de l'irrigation est l'élimination de divers microorganismes, la lubrification des instruments endodontiques insérés dans le canal et la dissolution des débris organiques et minéraux. Elle consiste à injecter abondamment dans le canal une solution d'irrigation telle que l'hypochlorite de sodium (NaOCl).

L'hypochlorite de sodium a une action antimicrobienne et de dissolution des tissus dont l'efficacité augmente avec la concentration, mais présente un risque de toxicité, notamment de cytotoxicité, à fortes concentrations.

Des concentrations « médicales » de NaOCl allant de 0,5% à 5,25% sont largement utilisées dans la pratique clinique. Bien que des solutions moins concentrées aient montré une certaine efficacité antimicrobienne, les concentrations susmentionnées présentent un effet bactéricide plus rapide et plus important, mais ne doivent en général pas être dépassées.

À trop forte concentration, l'hypochlorite de sodium a un effet extrêmement toxique sur les tissus vitaux, c'est un liquide corrosif. Propulsé en quantité importante dans le péri-apex, il peut provoquer des effets tels que des hémolyses, une ulcération de la peau, une nécrose, voire une faiblesse des nerfs faciaux de la région concernée. De plus, l'hypochlorite de sodium a un effet sur la dureté dentinaire. Une exposition prolongée à une forte concentration peut diminuer le module d'élasticité (en flexion) de 1a dentine.

Il est donc capital de connaitre avec précision la concentration de la solution d'irrigation, en l'occurrence de NaOCl, dans le canal, surtout dans la zone apicale, pour assurer un traitement efficace et limiter tout risque de toxicité.

Aucun localisateur d'apex ou analogue, à la connaissance du demandeur, ne permet de déterminer cette concentration dans la zone apicale, en particulier au niveau de la jonction cémento-dentinaire.

JP 2007 229110 A divulgue un dispositif de localisation électronique d'apex dans un canal radiculaire d'une dent, apte à être relié électriquement à un instrument endodontique s'engageant dans le canal et à une électrode labiale, et comportant un écran pour afficher une progression de l'instrument dans ledit canal relativement à un repère apical critique tel que la jonction cémento-dentinaire (JCD).

## Présentation de l'invention

La présente invention vise à répondre au besoin des praticiens de connaitre de façon pratique et fiable la concentration de la solution d'irrigation dans le canal radiculaire lors d'un traitement endodontique.

À cet effet, la présente invention a pour objet un dispositif de localisation électronique d'apex dans un canal radiculaire d'une dent, apte à être relié électriquement à un instrument endodontique s'engageant dans le canal et à une électrode labiale, et comportant un écran pour afficher une progression de l'instrument dans ledit canal relativement à un repère apical critique qui est la jonction cémento-dentinaire. Ce dispositif est remarquable en ce qu'il est en outre apte à mesurer par des moyens de traitement et de calcul, au voisinage dudit repère, par conductimétrie, une concentration d'une solution d'irrigation électrolytique injectée dans le canal radiculaire.

Selon un aspect de l'invention particulièrement avantageux, toute mesure de concentration de la solution d'irrigation est affichée automatiquement sur l'écran dès que l'instrument endodontique est détecté comme se trouvant dans le dernier millimètre avant la jonction cémento-dentinaire suivant le sens d'engagement de l'instrument endodontique dans le canal radiculaire.

Selon un mode de réalisation, un niveau de concentration mesurée, relativement à une plage de concentrations médicales, est affiché à l'écran du dispositif via un indicateur visuel.

Par exemple, l'indicateur visuel est un bargraphe.

En outre, le dispositif comporte des cordons de liaison par lesquels il peut être relié électriquement à l'instrument endodontique, à l'électrode labiale et analogues.

Avantageusement, des moyens de traitement et de calcul du dispositif sont adaptés à un calcul conductimétrique au moins pour une solution d'irrigation d'hypochlorite de sodium NaOCl, et permettent d'afficher un niveau de concentration mesurée sur une échelle comprenant au moins une valeur sensiblement égale à : 0,5%, 2,5% ou 5,25%, ces concentrations étant les plus recommandées et utilisées en endodontie, notamment lors des préparations canalaires.

Selon un mode de réalisation, l'écran affiche une information de validation de type « OK » tant que la concentration mesurée de la solution d'irrigation est sensiblement égale à une concentration d'injection de ladite solution.

Un procédé de mesure de concentration d'une solution d'irrigation dans un canal radiculaire d'une dent mettant en oeuvre un dispositif tel que présenté est également décrit. Le procédé ne fait pas partie de l'invention revendiquée.

De façon avantageuse, le procédé comprend des étapes de mise en mémoire des informations de concentration pour faciliter le travail du praticien car il peut lire la valeur de la concentration de la solution d'irrigation même lorsque l'instrument est sorti du canal du patient.

Les concepts fondamentaux de l'invention venant d'être exposés dans leur forme la plus élémentaire, d'autres détails et caractéristiques ressortiront plus clairement à la lecture de la description qui suit et en regard des dessins annexés, donnant à titre d'exemple non limitatif un mode de réalisation d'un dispositif conforme aux principes de l'invention.

## Présentation des dessins

Les figures sont données à titre purement illustratif pour l'intelligence de l'invention et ne limitent pas la portée de celle-ci. Les différents éléments sont représentés de manière schématique et ne sont pas nécessairement à la même échelle.

Il est ainsi illustré en :

[Fig. 1] : (déjà citée) une coupe longitudinale d'une racine dentaire au niveau de la zone apicale selon le concept apical de Kuttler ;

[Fig. 2] : un exemple d'utilisation d'un dispositif de localisation d'apex selon l'invention sur une dent pour mesurer la concentration de la solution d'irrigation ;

[Fig. 3] : le principe de mesure conductimétrique de la concentration de la solution d'irrigation au voisinage de la jonction cémento-dentinaire ;

[Fig. 4] : un montage électrique simplifié permettant de mesurer la concentration par conductimétrie selon l'invention ;

[Fig. 5a] : un exemple d'interface graphique de suivi de la progression de l'instrument dans le canal et de la concentration de la solution d'irrigation, selon l'état d'avancement de la préparation canalaire, ici l'instrument est en dehors de la zone de mesure au voisinage du repère apical critique ;

[Fig. 5b] : l'interface graphique lorsque l'instrument franchit le dernier millimètre avant la jonction cémento-dentinaire ;

[Fig. 5c] : l'interface graphique lorsque l'instrument est au niveau de l'apex.

## Description détaillée de modes de réalisation

Il convient de noter que certains dispositifs et procédés bien connus de l'homme du métier sont ici décrits pour éviter toute insuffisance ou ambiguïté dans la compréhension de la présente invention.

Dans le mode de réalisation décrit ci-après, on fait référence à un dispositif de localisation d'apex perfectionné pour mesurer la concentration de la solution d'irrigation dans le canal radiculaire d'une dent, destiné principalement à mesurer ladite concentration au voisinage immédiat de la jonction cémento-dentinaire lors d'une préparation canalaire. Cet exemple, non limitatif, est donné pour une meilleure compréhension de l'invention et n'exclut pas une autre utilisation du dispositif. Par exemple, le dispositif peut être adapté pour mesurer la concentration d'une solution d'irrigation ou d'un liquide physiologique dans une autre région anatomique dont le traitement est analogue au traitement endodontique, au moins en ce qui concerne l'injection d'une solution ionique dans un canal se terminant par une pointe (apex). Dans la suite de la description, la jonction cémento-dentinaire est désignée par son sigle JCD ; le terme « irriguant » désigne une solution d'irrigation ; et le terme « localisateur d'apex » désigne par extension un localisateur électronique d'apex. Tout renvoi à une partie anatomique de la racine dentaire au niveau de la zone apicale sera fait en référence à la figure 1 déjà décrite.

La figure 2 représente un localisateur d'apex 10 branché de façon conventionnelle sur un spécimen de dent T, ou sur un modèle de canal dentaire, par l'intermédiaire d'un instrument endodontique 20, tel qu'une lime, et d'un crochet labial 30, présentant tous deux des parties électriquement conductrices reliées au localisateur d'apex par des cordons de liaison 12 et 13 de sorte à former deux électrodes de mesure.

Selon un principe connu, le localisateur d'apex 10 permet de localiser l'apex dans le canal radiculaire CR de la dent T en se basant sur une mesure indirecte de résistance, ou plus généralement d'impédance, par application de la loi d'Ohm entre les bornes définies par les deux électrodes.

En règle générale, les localisateurs d'apex exploitent le constat selon lequel le canal radiculaire présente une impédance électrique fixe au niveau de la constriction apicale, et fonctionnent avec un courant électrique alternatif en circuit fermé grâce aux deux électrodes délimitant un ensemble anatomique conducteur entre le canal et la lèvre du patient. La tension électrique étant connue, imposée par le générateur du localisateur d'apex, ce dernier calcule l'intensité du courant circulant, celle-ci varie en fonction de l'impédance de l'ensemble anatomique. Ainsi, lors du franchissement de la zone apicale par l'instrument utilisé, la valeur de l'impédance change brusquement pour atteindre une valeur sensiblement constante d'un individu à l'autre, et le localisateur détecte l'intensité du courant correspondante.

Le localisateur d'apex 10 selon la présente invention exploite ce principe et le lien entre impédance et conductance électriques pour déterminer la concentration d'une solution d'irrigation dans le canal par conductimétrie, comme expliqué plus loin.

Le localisateur d'apex 10, selon l'exemple illustré, comprend un écran d'affichage 15 sur lequel différentes informations peuvent être lues pour permettre un suivi en temps réel de la pénétration de l'instrument endodontique 20 dans le canal radiculaire CR, et ce, jusqu'à la détection de l'apex.

Les cordons de liaison 12 et 13 du localisateur d'apex 10 sont pourvus à leur extrémité libre d'une pince 121 ou de tout autre moyen de connexion 131 pour s'accrocher aux parties électriquement conductrices de l'instrument endodontique 20 et du crochet labial 30.

L'instrument endodontique 20, selon l'exemple illustré, est une lime de préparation canalaire comportant une tige métallique 21 se terminant par une pointe 211. Une telle lime est de préférence souple pour s'adapter à des canaux radiculaires de différentes courbures. La pince 121 du cordon de liaison 12 vient se fixer sur la tige métallique 21 de la lime 20.

Le crochet labial 30 est quant à lui conformé pour assurer un contact avec la lèvre du patient et/ou sa gencive G.

De ce fait, l'instrument endodontique 20 et le crochet labial 30 délimitent électriquement un ensemble anatomique traversé par le courant électrique circulant, représenté par un trait interrompu S sur la figure 2.

Selon un aspect fondamental de la présente invention, le localisateur d'apex 10, grâce à ce montage conventionnel, permet en outre de mesurer la concentration de la solution d'irrigation dans le canal CR, au niveau de la pointe 211 de l'instrument 20, par une technique de conductimétrie.

En effet, la solution d'irrigation est une solution électrolytique dans laquelle baignent des porteurs de charge (ions) sous forme aqueuse produits par la réaction chimique de la solution d'irrigation en milieu canalaire.

On considère ici le cas de l'hypochlorite de sodium NaOCl qui reste actuellement l'irriguant le plus utilisé, voire incontournable, en endodontie.

L'effet antiseptique et dissolvant du NaOCl est obtenu par les réactions successives suivantes :

$$NaOCl + H_2O \leftrightarrow NaOH + HOCl \leftrightarrow Na^+ + OH^- + H^+ + OCl^-$$

La première est une réaction de dissolution de l'hypochlorite de sodium qui produit de l'acide hypochloreux HOCl. Ce dernier, en milieu basique, se dissocie lors de la deuxième réaction et produit ions hypochlorites OCl⁻ connus pour leur effet antibactérien. En effet, l'ion hypochlorite est un fort oxydant qui inhibe les enzymes bactériennes et conduit à une oxydation irréversible des groupes sulfhydriles (SH) qui constituent la plupart des membranes desdites enzymes, provoquant ainsi leur destruction.

Bien entendu, les ions formés après la deuxième réaction ci-dessus sont aqueux.

Du fait de la présence d'espèces chimiques électriquement chargées, la conductivité σ de 1a solution d'irrigation, dans le cas du NaOCl, s'écrit :

$$\sigma = \lambda_{Na^+}[Na^+] + \lambda_{OH^-}[OH^-] + \lambda_{H^+}[H^+] + \lambda_{OCl^-}[OCl^-]$$

Avec $\lambda_X$ la conductivité molaire ionique de l'ion X, et [X] sa concentration dans la solution.

En notant C la concentration de la solution à l'équilibre chimique, et au vu des coefficients de charge des ions en jeu, on peut écrire :

$$\sigma = C \sum_i \lambda_i$$

Or, on sait que la conductivité est proportionnelle à la conductance G qui n'est autre que l'inverse de la résistance R selon les formules :

$$\sigma = kG \ ; \ G = \frac{1}{R}$$

Le coefficient k dépend de la géométrie de la cellule de mesure.

En notant A la somme des conductivités molaires ioniques, on peut établir que :

$$C = \frac{\sigma}{\Lambda} = \frac{k}{\Lambda R} = \frac{k'}{R}$$

La concentration C est donc inversement proportionnelle à la résistance R, le coefficient de proportionnalité k' est une constante qui dépend essentiellement de la nature des ions dans la solution d'irrigation et de la géométrie de la cellule de mesure. La formulation simplifiée ci-dessus est donnée pour montrer le lien entre la résistance (ou l'impédance) de l'ensemble anatomique traversé par le courant électrique et la concentration de la solution d'irrigation, lien permettant d'utiliser le principe de base du localisateur d'apex pour déterminer ladite concentration par conductimétrie.

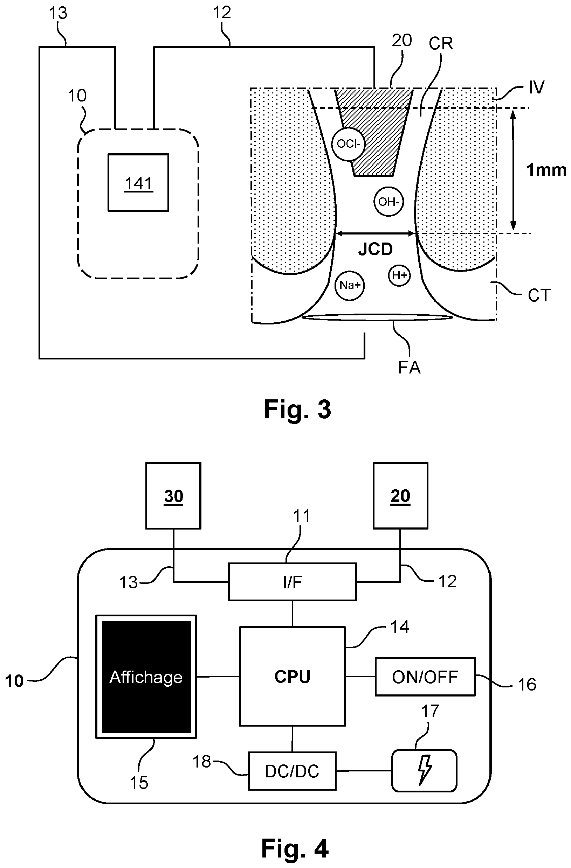

La figure 3 représente schématiquement le principe de mesure de la concentration de l'irriguant dans la zone apicale d'une racine dentaire dont la structure anatomique a été simplifiée pour une meilleure compréhension.

Lors de l'irrigation, la solution de NaOCl remplit le canal radiculaire CR jusqu'au foramen apical FA et pénètre même dans les moindres anfractuosités du canal grâce à sa faible tension superficielle.

L'objectif de la présente invention est de permettre au praticien de connaitre la concentration de l'irriguant au voisinage de la JCD, par exemple dans le dernier millimètre avant la JCD.

En règle générale, les nettoyages canalaires sont réalisés avec des concentrations de NaOCl entre 0,5%, 2,5% et 5,25%. Par conséquent, le localisateur d'apex 10 peut être calibré pour indiquer le niveau de concentration mesurée dans cette plage de concentrations dites « médicales ».

Parmi ses moyens de calcul et de traitement, le localisateur d'apex 10 peut comporter un module électronique spécifique 141 pour gérer les calculs conductimétriques indépendamment de la fonction de base dudit localisateur, à savoir la détection de l'apex. Un tel module pourrait être sous forme de carte électronique facilement intégrable à des modèles connus de localisateurs d'apex.

De préférence, les calculs conductimétriques sont directement opérés par un calculateur central du localisateur 10 comme expliqué ci-après en référence à la figure 4.

Du moment que le fonctionnement des localisateurs électroniques d'apex repose sur la variation d'un courant électrique en fonction de l'impédance propre du canal, et que celle-ci dépend directement de la concentration d'un éventuel électrolyte remplissant le canal, comme l'irriguant, la présente invention est adaptée aux différentes générations et technologies de localisateurs d'apex.

En particulier et selon l'exemple de réalisation de la figure 4, le localisateur d'apex 10 comporte une interface 11 de connexion avec les électrodes 20 et 30 par le biais des cordons de liaison 12 et 13, une unité de calcul 14 de type microprocesseur reliée aux différentes servitudes notamment le module d'affichage 15, un bouton marche/arrêt 16, une batterie électrique d'alimentation 17 ainsi qu'un régulateur de courant alternatif 18.

Le microprocesseur 14 opère à la fois les calculs de localisation d'apex et les calculs conductimétriques pour mesurer la concentration de la solution d'irrigation. Bien entendu, le localisateur d'apex dispose des moyens de mémoire nécessaires au stockage des informations générées.

Il est important de rappeler que la qualité d'un nettoyage canalaire dépend principalement de la concentration de l'irriguant (dans la plage médicale susmentionnée) et du temps de présence de l'irriguant dans le canal. Ainsi, le praticien a besoin de surveiller la concentration de l'irriguant dans le canal lorsqu'il opère un nettoyage canalaire par irrigation. D'autant plus que la connaissance de cette concentration au voisinage de la JCD est cruciale pour s'assurer de l'efficacité du traitement dans cette zone propice aux complications et à l'apparition d'abcès.

De ce fait, le localisateur d'apex 10 selon la présente invention permet un accès rapide et intuitif à l'information sur la concentration de l'irriguant dans la zone apicale du canal.

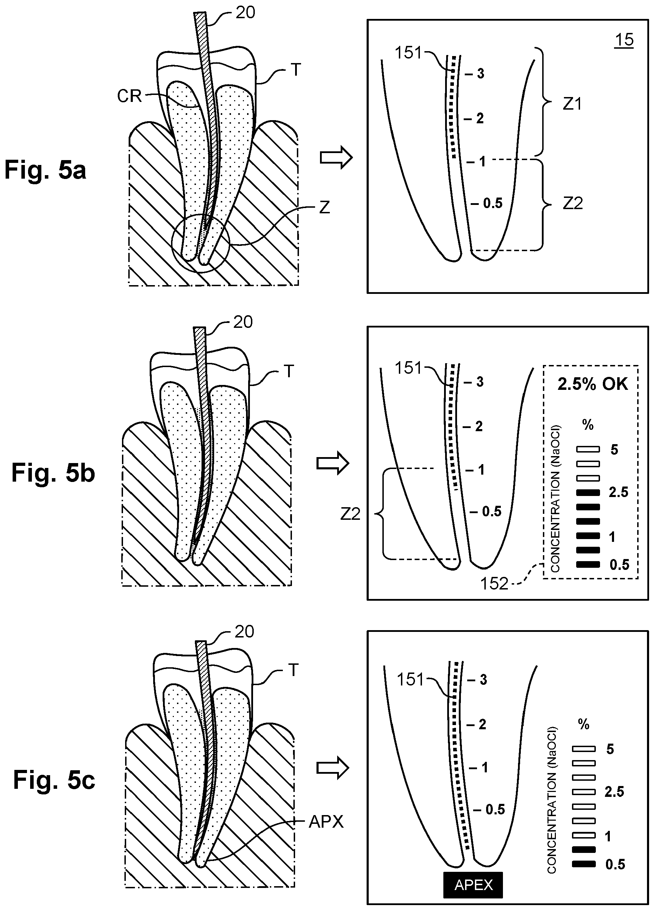

Les figures 5a à 5c donnent un exemple d'affichage (interface graphique) possible au fur et à mesure de la progression de l'instrument endodontique 20 dans le canal CR de la dent T. Cet affichage permet de visualiser, grossièrement, une section longitudinale partielle de la racine dentaire, au niveau de la zone apicale Z, sur laquelle une ligne de progression 151 en trait interrompu indique l'avancement de l'instrument 20 à l'intérieur du canal radiculaire CR. Selon l'exemple illustré, la zone apicale affichée correspond approximativement aux trois derniers millimètres de la racine, plus précisément aux trois derniers millimètres avant la JCD. L'échelle de représentation utilisée est de préférence dilatée sur le dernier millimètre pour un meilleur suivi dans cette zone critique du traitement endodontique.

Sur la figure 5a, l'instrument 20 commence à s'engager dans la zone apicale Z du canal radiculaire CR. L'affichage obtenu sur l'écran 15 du localisateur d'apex montre que la ligne de progression 151 n'est pas encore entrée dans le dernier millimètre avant la JCD qui correspond à la zone critique dans laquelle la concentration de l'irriguant doit être connue. En effet, on peut isoler deux zones sur la coupe de la racine affichée : une zone Z1 dans laquelle la mesure de la concentration n'est pas nécessaire et n'est donc pas effectuée, et une zone Z2 dans laquelle cette mesure est nécessaire pour ne pas laisser de doute au praticien lors de la préparation canalaire. La zone Z1 se situe en amont du dernier millimètre, tandis que la zone 2 correspond ce dernier millimètre avant la JCD. De plus, lors d'une injection de l'irriguant à concentration donnée, l'irriguant remplit sans difficulté la zone Z1 et sa concentration reste globalement constante, ce qui rend la mesure de ladite concentration inutile dans cette zone. En revanche, le remplissage de la zone Z2 au voisinage de la JCD (et donc souvent au voisinage de la constriction apicale) par la solution d'irrigation reste problématique et incertaine à cause de l'étroitesse du canal dans cette zone. D'où l'intérêt de mesurer la concentration dans cette zone critique.

Dans l'exemple des figures 5a à 5c, on considère une injection d'une solution d'hypochlorite de sodium NaOCl à une concentration de 2,5%.

La figure 5b montre que l'instrument 20 a franchi la zone apicale considérée de sorte que l'affichage obtenu indique la présence de la ligne de progression 151 dans la zone Z2 de mesure de la concentration. Le niveau de concentration mesurée est donc indiqué sur l'écran du localisateur d'apex via un indicateur visuel tel qu'un bargraphe 152. Par exemple, l'affichage du niveau de concentration peut apparaitre automatiquement et brusquement sur l'écran dès que l'instrument entre dans la zone de mesure Z2, afin d'avertir visuellement le praticien. Dans l'exemple illustré, l'écran indique un niveau de concentration de 2,5%, en accord avec la concentration d'injection. Un message de suivi tel que « OK » peut également être affiché lorsque la concentration est au niveau requis pour que le praticien utilisateur ne soit pas perturbé par une lecture compliquée de l'information. Ainsi, le praticien monopolise une majeure partie de sa vigilance dans la manipulation des instruments endodontiques à l'intérieur du canal.

En outre, la mise en mémoire de la valeur de concentration permettrait une lecture aisée même après avoir retiré l'instrument canalaire. La mise à zéro de l'information interviendrait lors de la réintroduction de l'instrument dans le canal ou de l'arrêt automatique du localisateur.

Sur la figure 5c, l'instrument 20 a atteint l'apex ou, tout du moins, en est très proche, moyennant des tolérances admissibles, et l'écran affiche une ligne de progression 151 s'étendant jusqu'au « zéro » du repère millimétrique. Le localisateur d'apex peut signaler la proximité de l'instrument 20 de l'apex par une notification visuelle et/ou sonore. Selon l'exemple illustré, la concentration de l'irriguant est inférieure à 1% et reste donc en deçà de la valeur requise. Dans ce cas, le praticien doit renouveler l'irrigation du canal en ajoutant une certaine quantité de solution pour augmenter la concentration de l'irriguant dans cette zone. Par ailleurs, l'écran n'affiche aucun message négatif lorsque la concentration n'est pas suffisante pour éviter toute confusion et rendre l'interface graphique la plus intuitive possible.

Toutefois, l'exemple simplifié de l'interface graphique présenté ci-dessus n'est pas limitatif.

**Revendications**

1. Dispositif (10) de localisation électronique d'apex dans un canal radiculaire (CR) d'une dent (T), apte à être relié électriquement, au moyen de cordons de liaison (12, 13), à un instrument endodontique (20) pouvant s'engager dans le
canal et à une électrode labiale (30), et comportant un écran (15) pour afficher une progression (151) de l'instrument dans ledit canal relativement à un repère apical critique qui est la jonction cémento-dentinaire (JCD), **caractérisé en ce qu'**il est en outre apte à mesurer par des moyens de traitement et de calcul (14), au voisinage dudit repère, par conductimétrie, une concentration d'une solution d'irrigation électrolytique injectée dans le canal radiculaire.

2. Dispositif selon la revendication 1, dans lequel toute mesure de concentration de la solution d'irrigation est affichée automatiquement sur l'écran dès que l'instrument endodontique (20) est détecté comme se trouvant dans le dernier millimètre avant la jonction cémento-dentinaire (JCD) suivant le sens d'engagement de l'instrument endodontique (20) dans le canal radiculaire (CR).

3. Dispositif selon l'une des revendications 1 ou 2, dans lequel un niveau de concentration mesurée, relativement à une plage de concentrations médicales, est affiché à l'écran (15) via un indicateur visuel (152).

4. Dispositif selon la revendication 3, dans lequel l'indicateur visuel (152) est un bargraphe.

5. Dispositif selon l'une quelconque des revendications précédentes, dans lequel des moyens de traitement et de calcul (12) sont adaptés à un calcul conductimétrique au moins pour une solution d'irrigation d'hypochlorite de sodium (NaOCl), et permettent d'afficher un niveau de concentration mesurée sur une échelle comprenant au moins une valeur sensiblement égale à : 0,5%, 2,5% ou 5,25%.

6. Dispositif selon l'une quelconque des revendications précédentes, dans lequel l'écran (15) affiche une information de validation tant que la concentration mesurée de la solution d'irrigation est sensiblement égale à une concentration d'injection de ladite solution.

**Patentansprüche**

1. Vorrichtung (10) zur elektronischen Lokalisierung eines Apex in einem Wurzelkanal (CR) eines Zahns (T), die geeignet ist, mittels Verbindungskabeln (12, 13) elektrisch mit einem Endodontie-Instrument (20), das in den Kanal einrücken kann, und mit einer Labialelektrode (30) verbunden zu werden, und einen Bildschirm (15) umfasst, um ein Vordringen (151) des Instruments im Kanal relativ zu einer kritischen apikalen Markierung, bei der es sich um die Zement-Dentin-Grenze (JCD) handelt, anzuzeigen, **dadurch gekennzeichnet, dass** sie weiter geeignet ist, über Verarbeitungs- und Rechenmittel (14) durch Leitfähigkeitsmessung in der Nähe der Markierung eine Konzentration einer elektrolytischen Spüllösung, die in den Wurzelkanal injiziert wird, zu messen.

2. Vorrichtung nach Anspruch 1, wobei jede Messung der Konzentration der Spüllösung automatisch auf dem Bildschirm angezeigt wird, sobald erkannt wird, dass sich das Endodontie-Instrument (20) in der Einrückrichtung des Endodontie-Instruments (20) im Wurzelkanal (CR) im letzten Millimeter vor der Zement-Dentin-Grenze (JCD) befindet.

3. Vorrichtung nach einem der Ansprüche 1 oder 2, wobei ein gemessenes Konzentrationsniveau relativ zu einem Bereich medizinischer Konzentrationen über einen visuellen Indikator (152) auf dem Bildschirm (15) angezeigt wird.

4. Vorrichtung nach Anspruch 3, wobei der visuelle Indikator (152) ein Balkendiagramm ist.

5. Vorrichtung nach einem der vorstehenden Ansprüche, wobei Verarbeitungs- und Rechenmittel (12) für eine leitfähigkeitsmesstechnische Berechnung mindestens für eine Natriumhypochlorit- (NaOCl-) Spüllösung geeignet sind und es ermöglichen, ein gemessenes Konzentrationsniveau auf einer Skala anzuzeigen, die mindestens einen Wert von im Wesentlichen gleich: 0,5 %, 2,5 % oder 5,25 % umfasst.

6. Vorrichtung nach einem der vorstehenden Ansprüche, wobei der Bildschirm (15) eine Validierungsinformation anzeigt, solange die gemessene Konzentration der Spüllösung im Wesentlichen gleich einer Injektionskonzentration der Lösung ist.

**Claims**

1. A device (10) to electronically locate the apex in a root canal (CR) of a tooth (T), capable of being electrically connected, by means of connecting cords (12, 13), to an endodontic instrument (20) capable of engaging in the canal and a labial electrode (30), and including a screen (15) to display a progression (151) of the instrument in said canal relative to a critical apical point of reference which is the cemento-dentinal junction (CDJ), **characterised in that** it is further capable of measuring by processing and calculation means (14), in the vicinity of said point of reference, by conductimetry, a concentration of an electrolyte irrigation solution injected into the root canal.

2. The device according to claim 1, wherein any concentration measurement of the irrigation solution is automatically displayed on the screen as soon as the endodontic instrument (20) is detected as being in the last millimetre before the cemento-dentinal junction (CDJ) following the direction of engagement of the endodontic instrument (20) in the root canal (CR).

3. The device according to one of claims 1 or 2, wherein a measured concentration level, relative to a range of medical concentrations, is displayed on the screen (15) via a visual indicator (152).

4. The device according to claim 3, wherein the visual indicator (152) is a bar graph.

5. The device according to any one of the preceding claims, wherein processing and calculation means (12) are adapted to a conductometric calculation at least for an irrigation solution of sodium hypochlorite (NaOCl), and allow to display a concentration level measured on a scale comprising at least one value substantially equal to: 0.5%, 2.5% or 5.25%.

6. The device according to any one of the preceding claims, wherein the screen (15) displays validation information as long as the measured concentration of the irrigation solution is substantially equal to an injection concentration of said solution.

Fig. 1

Fig. 2

**Fig. 3**

**Fig. 4**

Fig. 5a

Fig. 5b

Fig. 5c

**RÉFÉRENCES CITÉES DANS LA DESCRIPTION**

**Documents brevets cités dans la description**

- JP 2007229110 A